# NEUE EUROPÄISCHE PATENTSCHRIFT

(11) **EP 1 464 306 B2**
(45) Veröffentlichungstag und Bekanntmachung des Hinweises auf die Entscheidung über den Einspruch: **11.06.2014**
(45) Hinweis auf die Patenterteilung: 11.04.2007
(21) Anmeldenummer: 04007507.9
(22) Anmeldetag: 20.09.1994
(51) Int. Cl.: A61F 2/44, A61F 2/46

(54) **Implantat für den Zwischenwirbelraum**
Implant for the intervertebral space
Implant pour l'espace intervertébral

(30) Priorität: 21.09.1993 BE 9300982
(43) Veröffentlichungstag der Anmeldung: 06.10.2004
(62) Teilanmeldung aus: 94926078.0
(73) Patentinhaber: Synthes GmbH, 4436 Oberdorf (CH)
(72) Erfinder: Beckers, Louis Francois Charles, B-2820 Rijmenam (BE); Schläpfer, Johannes Fridolin, CH-8750 Glarus (CH)
(74) Vertreter: Lusuardi, Werther

(56) Entgegenhaltungen:
- EP-A- 0 260 044
- EP-A- 0 307 241
- EP-A- 0 493 698
- EP-A1- 0 317 972
- EP-A1- 0 346 129
- EP-A1- 0 392 076
- EP-A1- 0 637 440
- EP-A2- 0 307 241
- EP-B1- 1 464 306
- WO-A-89/12431
- WO-A-90/00037
- WO-A-92/14423
- WO-A-93/01771
- WO-A1-90/00037
- BE-A- 0 930 082
- US-A- 3 486 505
- US-A- 4 097 935
- US-A- 4 349 921
- US-A- 4 349 921
- US-A- 4 770 860
- US-A- 4 772 287
- US-A- 5 092 893
- US-A- 5 192 327
- SPRINGER NEW YORK: 'Medical ceramics based on hydroxyl-apatite' JOURNAL OF GLASS AND CERAMICS Bd. 48, Nr. 7, Juli 1991, Seiten 319 - 321
- A. A. WHITE ET AL.: 'Clinical Biomechanics of the Spine', Bd. 6, 1990, LIPPINCOTT WILLIAMS & WILKINGS, PHILADELPHIA Seiten 4 - 5
- Vertebral Morphology in the Mature Human Spine
- J. L. BERRY ET AL.: 'A Morphometric Sutdy of Human Lumbar and Selected Thoracic Vertebrae' STUDIE OF SELECTED VERTEBRAE Bd. 12, Nr. 4, 1987, Seiten 364 - 366
- P. V. SCOLES ET A.: 'Vertebral Body and Posterior Element Morphology: The Normal Spine in Middle Life' SPINE Bd. 13, Nr. 10, 1988, Seiten 1082 - 1086
- PANJABI ET AL.: 'Quantitative Three-Dimensional Anatomy' ANATOMY OF HUMAN LUMBAR VERTEBRAE Seiten 299 - 301 - 305 - 3
- P. M. LIN ET AL.: 'Lumbar Interbody Fusion', 1989, ASPEN PUBLISHERS, INC., UNITED STATES OF AMERICA Seiten 180 - 184
- PR. J.-L. HUSSON ET AL.: 'Restabilisation Intersomatique Du Rachis Lombaire, Arthodese Inter-Somatique Lombaire par Voie Posterierure Par Cages Ayze - Description Technique Chirurgicale:Revue Clinique et Radiologique A Propos De 47 Cas.', 1996, SAURAMPS MÉDICAL, MONTPELLIER Seiten 160 - 171
- Vetebral Space
- 'Luminary PLIF Spacer' SYNTHES SPINE 2006,

## Beschreibung

Die Erfindung bezieht sich auf ein Implantat für den Zwischenwirbelraum gemäss dem Oberbegriff des Patentanspruchs 1. Solche Implantate sind hauptsächlich dazu bestimmt, Knochenbrücken an Wirbelkörpern zu fördern und welche nach der Resektion von Diskus bzw. Zwischenwirbelscheibe zwischen Wirbelkörper und Rückgrat angebracht werden.

Ein Implantat gemäss dem Oberbegriff des Anspruchs 1 ist in der WO 90/00037 MICHELSON offenbart.

Es ist bekannt, dass bei Beschädigung einer Zwischenwirbelscheibe diese entfernt und der entstandene Raum mit kortiko- spongiösem Knochen gefüllt werden kann.

Bei dieser Methode werden die Wirbelkörper zuerst weitmöglichst mit Hilfe von Spreizern auseinandergedehnt. Eine Spezialtechnik besteht darin, dass keilförmige Elemente - sogenannte Dilatatoren - zwischen die beiden Wirbelkörper eingeführt werden, um sie schrittweise auseinander zu dehnen. Dabei wird abwechselnd links und rechts jeweils ein Dilatator mit einem 1 mm grösseren Durchmesser von posterior angebracht. Nachdem die grösstmögliche Dehnung erreicht ist, werden die Dilatatoren durch den obengenannten kortiko-spongiösen Knochen ersetzt.

Diese bekannte Technik hat den Nachteil, dass der Knochen schwierig zu handhaben und in die richtige Position zu bringen ist, wobei Korrekturen nahezu ausgeschlossen sind. Ein weiterer Nachteil dieser Technik besteht darin, dass im Zwischenwirbelraum eine rechteckige oder zylinderförmige Aussparung ausgestochen und/oder ausgefräst werden muss, um die Knochen pfropfen zwischen die ursprünglich konkaven Seiten der angrenzenden Wirbelkörper bringen zu können, was umständlich ist und zusätzlich zur Beschädigung der Wirbelkörper führt.

Hier will die Erfindung Abhilfe schaffen. Der Erfindung liegt die Aufgabe zugrunde, ein Implantat für den Zwischenwirbelraum zu schaffen, welches aufgrund seiner spezifischen Form und der Einbringungsart eine äusserst stabile Verklemmung zwischen den Wirbelkörpern ermöglicht, ohne dass dabei die Oberfläche der knöchernen Deckplatte der Wirbelkörper beschädigt wird.

Eine weitere Aufgabe der Erfindung liegt in der Schaffung eines Implantates für den Zwischenwirbelraum zu schaffen, welches ohne Verwendung von Dilatatoren eingebracht werden kann.

Die Erfindung löst die gestellte Aufgabe mit einem Implantat für den Zwischenwirbelraum, welches die Merkmale des Anspruchs 1 aufweist.

Weitere vorteilhafte Ausgestaltungen der Erfindung sind in den abhängigen Ansprüchen gekennzeichnet.

Da das erfindungsgemässe Implantat mit einer Vorrichtung zur Ergreifung durch ein Werkzeug versehen ist, kann relativ mühelos eine externe Kraft darauf ausgeübt werden, die es ermöglicht, das Implantat nach der Anbringung zu bewegen oder es eventuell wieder herauszunehmen.

Die Vorrichtungen zur Ergreifung durch ein Werkzeug können als Ansatzpunkte derart gestaltet sein, dass eine Rotationskraft und/oder eine axiale Kraft und/oder eine seitliche Kraft auf das Implantat ausgeübt werden kann

Bei einer vorteilhaften Ausführungsform sind diese Ansatzpunkte zumindest so gestaltet, dass sie die Ausübung einer Rötationskraft auf das Implantat ermöglichen, wobei das Implantat dabei unterschiedliche Querschnittslängen aufweisen muss, damit es durch die Drehung des Implantats mehr oder weniger eingeklemmt wird oder selbst in einer Position völlig lose sitzen und somit mühelos zwischen die Wirbelkörper gebracht werden kann und in einer anderen Position die erforderliche Einklemmung aufweist.

Der Körper des Implantats weist in einer Ebene ein linsenförmig zugeschnittenes Profil auf, das grösstenteils mit der bikonkaven Form der sagittalen Schnittfläche des Zwischenwirbelraums übereinstimmt, wobei derselbe Körper in der anderen Ebene hauptsächlich parallele, flache oder nur leicht gebogene Seiten und ein abgerundetes Ende aufweist, damit er in den Zwischenwirbelraum gedrückt werden kann, ohne eine Aussparung in den Wirbelkörper stechen zu müssen und ohne die Umrandung des Wirbelkörpers zu beschädigen.

Das Implantat ist vorzugsweise hohl, damit es mit Knochenmaterial gefüllt werden kann.

Um die Erfindung besser zu verdeutlichen, werden nachstehend einige Beispiele vorteilhafter Ausführungsformen - auf die sich die Erfindung jedoch nicht beschränkt - mit Verweisen nach den entsprechenden Zeichnungen beschrieben.

Es zeigen:
Fig. 1 eine schematische Darstellung zweier Wirbelkörper, die mit zwei Dilatatoren auseinandergedehnt sind;
Fig. 2 einen Querschnitt entlang der Linie II-II in Fig. 1, wobei ein Dilatator durch einen kleinen Knochenquader ersetzt ist;
Fig. 3 eine perspektivische Darstellung eines erfindungsgemässen Implantats mit einem dazu verwendbaren Werkzeug;
Fig. 4 eine Aufsicht in Richtung des Pfeiles F4 der Fig. 3;
Fig. 5 eine Aufsicht in Richtung des Pfeiles F5 der Fig. 3;
Fig. 6 einen Querschnitt entlang der Linie VI-VI in Fig. 4;
Fig. 7 eine schematische Darstellung des Implantats nach Fig. 3 nach erfolgter Einführung zwischen zwei Wirbelkörpern;
Fig. 8 eine schematische Darstellung des Implantats nach Fig. 3 nach erfolgter Einführung zwischen zwei Wirbelkörpern und Rotation um 90°;
Fig. 9 eine schematische Darstellung einer weiteren Ausführungsform der Erfindung mit einem dazu verwendbaren Werkzeug;
Fig. 10 einen Querschnitt durch ein erfindungsgemässes Implantat mit einseitiger Abrundung;
Fig. 11 einen Querschnitt durch ein erfindungsgemässes Implantat mit doppelseitiger Abrundung über die Diagonale;
Fig. 12 einen Querschnitt durch eine paarige Anordnung zweier spiegelsymmetrischer erfindungsgemässer Implantate;
Fig. 13 eine schematische Darstellung paarig angeordneter, spiegelsymmetrischer erfindungsgemässer Implantate mit deren Hilfe der Bandscheibenraum distrahiert werden kann;
Fig. 14 eine schematische Darstellung von zwei flach im Bandscheibenraum liegenden Implantaten, die über ein drittes tmptantatanterior verbunden sind, vor und nach der Rotation in die Konkavität der Deckplatten der angrenzenden Wirbelkörper;
Fig. 15 eine perspektivische Darstellung eines Implantates mit einem Längsschnitt zur Aufnahme von spongiösen Knochenmaterial oder osteokonduktives bzw. osteoinduktives Material und einer Querperforation der Wände für das Knochenwachstum;
Fig. 16 eine perspektivische Darstellung eines Implantates mit längsstrukturierten Kontaktflächen zwischen Implantat und Knochen, wobei die Längsstrukturierung derart gestaltet ist, dass das Rotieren des Implantates in die Konkavität der Deckplatten nur in einer Richtung möglich ist;
Fig. 17 eine perspektivische Darstellung eines Implantates mit querstrukturierten Kontaktflächen zwischen Implantat und Knochen, wobei die Querstrukturierung derart gestaltet ist, dass die eine Strukturierung eine Translation in anteriorer und die andere eine Translation in posteriorer Richtung verhindert. Die Verhinderung der Translation in anteriorer Richtung führt zu einer Entlastung des verbliebenen Annulus, der nach jüngster Forschung innerviert ist und damit auf anterioren Druck mit Schmerzsignalen reagieren könnte.

Die Erfindung und Weiterbildungen der Erfindung werden im folgenden anhand der teilweise schematischen Darstellungen mehrerer Ausführungsbeispiele noch näher erläutert.

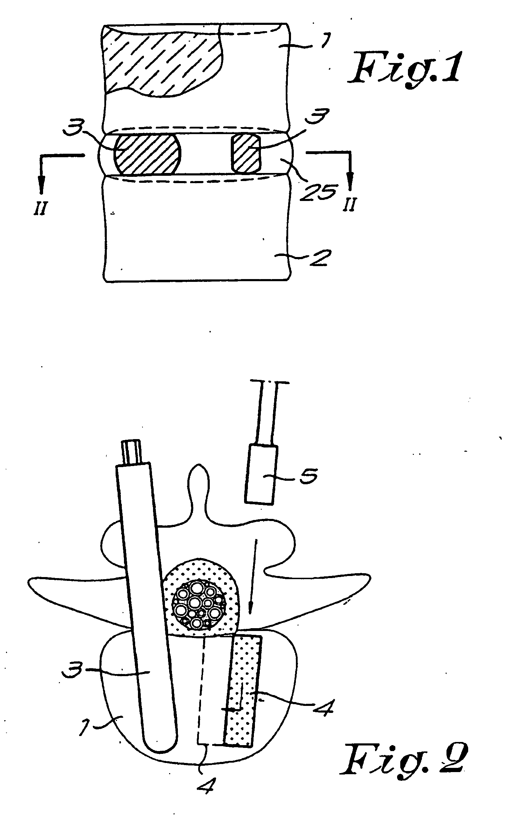

Anhand der Figuren 1 und 2 wird vorerst die bekannte Technik beschrieben. Wenn eine Zwischenwirbelscheibe entnommen wird, werden, wie in Fig. 1 dargestellt, die zwei angrenzenden Wirbelkörper 1 und 2 soweit als möglich auseinandergedehnt, um die Dilatatoren 3 anbringen zu können. Nachdem die Wirbelkörper 1 und 2 sich im gewünschten Abstand befinden, werden die Dilatatoren 3, wie in Fig. 2 dargestellt, durch die Knochehpfropfen 4 ersetzt, die nach dem Ausstechen einer Aussparung in den Wirbelkörpern 1 und 2 mit einem Andruckelement 5 zwischen die Wirbelkörper gepfropft werden. Es ist ersichtlich, dass diese Technik die in der Beschreibungseinleitung genannten Nachteile aufweist.

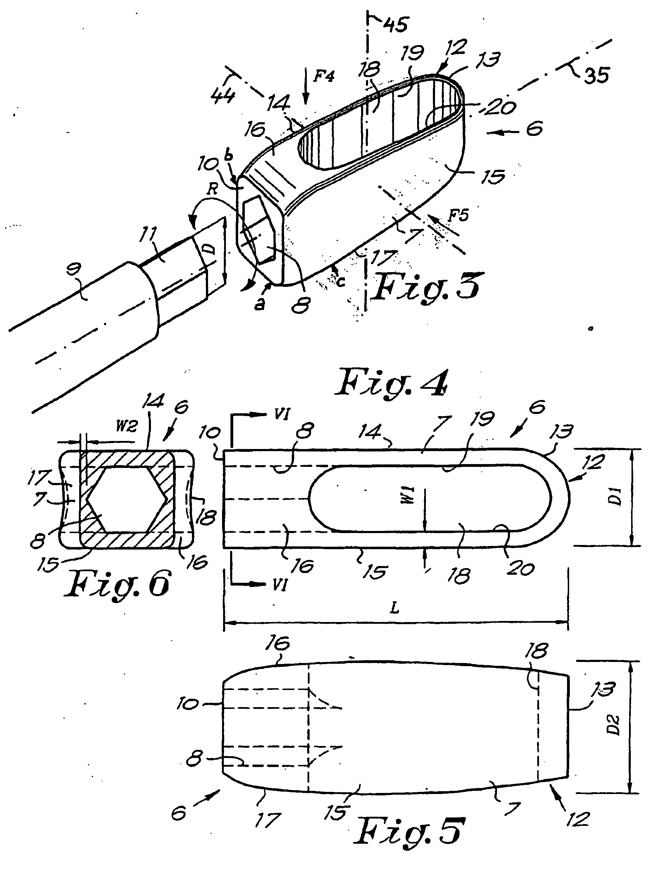

Das in den Fig. 3 - 6 dargestellte, erfindungsgemässe Implantat, überwindet nun diese Nachteile und erlaubt, dass es schnell eingebracht und zusätzlich, falls erforderlich, unter Kraftanwendung zwischen zwei Wirbelkörpern geklemmt werden kann. Das Implantat 6 besteht im wesentlichen aus einem Körper 7 mit einer Vorrichtung 8 zur Ergreifung durch ein Werkzeug 9.

Die Vorrichtungen 8 zur Ergreifung durch eine Werkzeug 9 sind so gestaltet, dass eine Rotationskraft, eine axiale Kraft und/oder eine seitliche Kraft auf das Implantat 6 ausgeübt werden kann, vorzugsweise in allen Richtungen.

Vorteilhafterweise sind, wie in den Fig. 3 - 6 dargestellt, die Vorrichtungen 8 derart gestaltet, dass darauf mindestens eine Rotationskraft R ausgeübt werden kann und in Verbindung damit das Implantat so gestaltet ist, dass es verschiedene Durchmesser oder Querschnittslängen aufweist, damit durch Drehung an den genannten Vorrichtungen 8 der Körper 7 des Implantats 6 mit grossen oder kleinen Abständen zwischen die Wirbelkörper 1 und 2 gebracht werden kann.

Die Vorrichtungen 8 bestehen bei der Ausführungsform nach den Fig. 3 - 6 aus einer in der Innenseite des Körpers 7 angebrachten Aussparung am hinteren axialen Ende 10 des Implantats 6. Die Aussparung ermöglicht es, ein Werkzeug 9 einzuführen. Wie dargestellt, kann die Aussparung aus einer axialen mehrkantigen, z.B. sechskantigen Öffnung bestehen, wobei hierbei ein Werkzeug 9 verwendet werden muss, das mit einem sechskantigen Ende 11 in der Form eines Imbusschlüssels versehen ist.

Die Verwendung einer in der Innenseite angebrachten Öffnung für das Einführen eines Werkzeugs 9, also die vorgenannte Aussparung, bietet den Vorteil, dass am Implantat 6 keine störenden Elemente hervorstehen.

Der Körper 7 weist vorzugsweise eine besondere Form mit einem oder mehreren der hiernach aufgeführten Kennzeichen auf:
- das vordere axiale Ende 12 des Körpers 7 sollte abgerundet oder keilförmig ausgebildet sein, da dies die Einführung in den Zwischenwirbelraum 25 erleichtert;
- die Abrundung 13 am vorderen axialen Ende 12 des Körpers 7 verläuft vorzugsweise nur entlang einem Querschnitt parallel zum kleinsten Durchmesser D1
- siehe Fig. 4 - und nicht nach dem dazu im rechten Winkel stehenden Querschnitt, wie in Fig. 5 dargestellt.
- die Seitenflächen 14 und 15, durch die der kleinste Durchmesser verläuft, sind mit Ausnahme der Abrundung 13 vorzugsweise parallel und flach;
- bei Seitenansicht weist der Körper 7 wie in Fig. 5 dargestellt ein abgekantetes, linsenförmiges Profil auf, also ein Profil, das mit der natürlichen bikonkaven Form übereinstimmt, die ein Zwischenwirbelraum in der sagittalen Schnittfläche aufweist. Die Übergänge zwischen den Seitenflächen 14 und 15 und die Oberfläche 16 sowie die Unterfläche 17 sind abgerundet;
- die Ober- und Unterfläche 16;17 sind vorzugsweise entlang einem Querschnitt mindestens teilweise und besser vollkommen flach; die Tatsache, dass die Ober- und Unterfläche 16;17 in Querrichtung mindestens teilweise flach sind, bietet den Vorteil, dass sie in eingeklemmtem Zustand Kippstabilität bieten;
- der Körper 7 weist eine oder mehrere Öffnungen oder Aussparungen für die Füllung mit Pfropfmaterial auf; gemäss den Fig. 3 bis 6 besteht eine durchgehende, sich von der Oberfläche 16 bis zur Unterfläche 17 erstreckende Öffnung 18; die Öffnung 18 besteht aus einem länglichen Schlitz mit den parallelen Wänden 19 und 20; die vorgenannte Aussparung 8 kann sich dabei auf Wunsch bis in die Öffnung 18 erstrecken.
- Das Implantat wird vorzugsweise aus Titan oder einer für Implantate geeigneten Titanlegierung gefertigt.
- Die Öffnung 18 oder der Schlitz im Körper 7 des Implantats der Fig. 3 kann angebracht werden, wenn mehrere vertikale Bohrungen im Körper 7 gemacht und die Zwischenwände danach weggefräst werden;
- vorzugsweise weist das Implantat 6, und genauer gesagt der Körper 7 eine Länge L von ungefähr 22 mm auf und wird ausgehöhlt, bis ungefähr auf eine Wanddicke W1 von 1,5 mm bestehen bleibt. Das hintere axiale Ende 10 mit der Vorrichtung 8 weist vorzugsweise einen Mindestdurchmesser von 6 mm auf; um zu erreichen, dass die Mindestwanddicke W2 an der Stelle der Vorrichtung 8 und die Dicke D des Werkzeugs grösstmöglich ist, wird die vorgenannte Aussparung so angebracht, dass die Richtung ihres grössten Durchmessers mit der des grössten Durchmessers des Körpers 7 übereinstimmt.

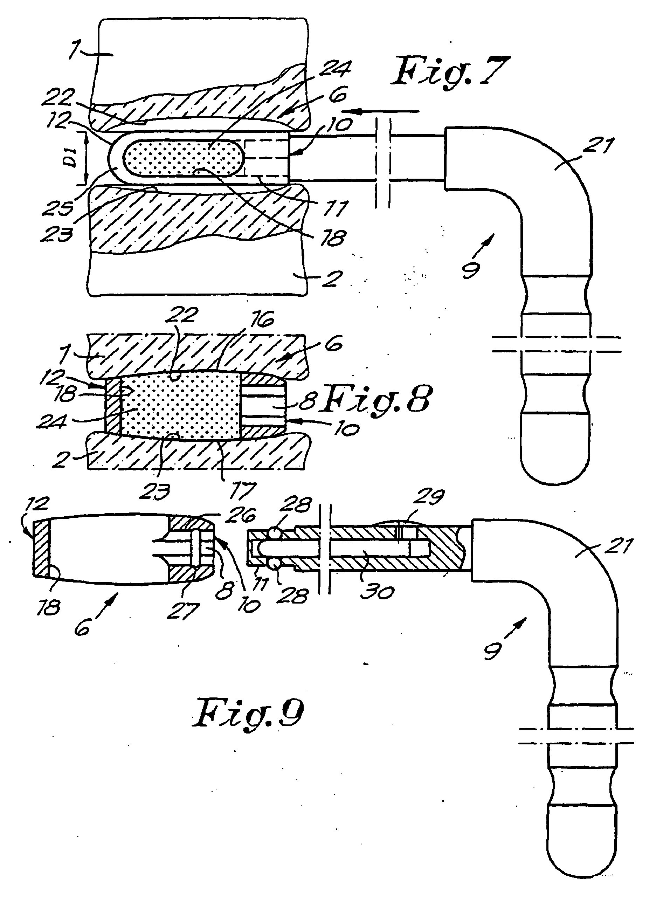

Anhand der Fig. 7 und 8 wird nun nachstehend die Verwendung und das Anbringen des Implantats 6 zwischen zwei Wirbelkörpern 1 und 2 beschrieben.
In Fig. 7 wird dargestellt, wie das Implantat 6 auf dem Ende eines einem Schlüssel ähnelnden entsprechenden Werkzeugs 9 zwischen den beiden Wirbelkörpern 1 und 2 angebracht werden kann. Das Implantat 6 wird dabei mit dem kleinsten Durchmesser D1 zwischen die zueinander gerichteten Seiten 22 und 23 der Wirbelkörper 1 und 2 eingeführt. Dabei ist es bereits mit Knochenpfropfen 24 gefüllt. Um das Implantat 6 danach passend oder klemmend zwischen den Wirbelkörpern 1 und 2 anzubringen, wird der Schlüssel 21 des Werkzeugs 9 um 90° gedreht, damit, nach Entfernung des Werkzeugs 9, ein wie in Fig. 8 dargestellter Zustand erreicht wird. Da die Knochenpfropfen 24 an die Wirbelkörper 1 und 2 anschliessen, kann das Implantat 6 durch Verwachsung der Knochenpfropfen 24 festen Halt erreichen.

Das Implantat 6 kann ohne besondere Hilfsmittel eingebracht werden, der Vorgang kann jedoch vereinfacht werden, wenn die Wirbel vorher mittels ovaler Dilatatoren an der linken und rechten Seite auseinandergedehnt und so lange in dieser Position beibehalten werden, bis an der anderen Seite ein Implantat 6 eingeklemmt ist. Da die Anwesenheit des Implantats 6 dann wiederum verhindert, dass sich die Wirbelflächen erneut zusammenschieben, kann jetzt der letzte Dilatator entfernt und eventuell durch ein zweites Implantat 6 ersetzt werden. Normalerweise müssen zwei Implantate 6 angebracht werden.

Aus den Fig. 7 und 8 wird deutlich ersichtlich, dass bei der Verwendung eines drehbaren Implantats 6 mit unterschiedlichen Durchmessern D1 und D2 dieses frei und ohne viel Mühe zwischen die Wirbelkörper 1 und 2 eingeführt werden kann und es andererseits durch Drehung in perfekten Halt zwischen die Wirbelkörper gebracht werden kann. Daher ist es auch nicht erforderlich, den Zwischenwirbelraum 25 für den Erhalt eines rechteckigen oder zylinderförmigen Raumes auszustechen oder auszufräsen.

Da der Körper 7 des Implantats 6 unterschiedliche Durchmesser D1 und D2 aufweist, ist er einfach aus dem Zwischenwirbelraum 25 zu entfernen. Es ist eindeutig, dass das Implantat 6 nach dem Einklemmen erneut gelöst werden kann, wenn es in entgegen gesetzte Richtung gedreht wird, bis sich der kleinste Durchmesser D1 zwischen den Wirbelkörpern 1 und 2 befindet.

Wenn ein Implantat 6 verwendet wird, das einen Körper 7 mit einer Form aufweist, die mit der natürlichen bikonkaven Form des Zwischenwirbelraums 25 übereinstimmt, entsteht automatisch ein perfekter Anschluss zwischen den Seiten 22 und 23 der Wirbelkörper 1 und 2 und der Oberfläche 16 sowie der Unterfläche 17 des mit Knochenpfropfen 24 verpfropften Implantats 6.

Die Technik des Drehens des Implantates 6 hat folgende Vorteile:
- Wenn die Deckplatten konkav gewölbt sind, dann bringt das Rotieren die Möglichkeit, das Implantat 6 derart zu gestalten, dass es in einer Dimension flach ist und in der anderen Dimension der Geometrie der Deckplatte entspricht. Die flache Dimension erleichtert das Einschieben von posterior; die gewölbte Fläche ergibt einen optimalen Kontakt mit den Deckplatten;
- wenn die Deckplatten flach sind, dann kann das Rotieren benutzt werden, um den Bandscheibenraum zu spreizen;
- eine Querverzahnung der Oberfläche des Implantates ist möglich, da das Implantat erst nach der Insertion gedreht wird.

Selbstverständlich kann das Implantat 6 in verschiedenen Formen verwirklicht werden. Anstelle einer Aussparung für einen Sechskantimbusschlüssel können auch andere Aussparungsformen verwendet werden, die z.B. aus vierkantigen, rechteckigen oder ovalen Öffnungen bestehen.

Obwohl die Vorrichtungen 8 zur Ergreifung durch ein Werkzeug 9 vorzugsweise in der Innenseite des Implantats 4 angebracht sind, ist dies nicht unbedingt erforderlich. Sie können auch aus einem vorstehenden Teil oder aus einer bestimmten Formgebung des hinteren axialen Endes 10 bestehen, so dass der vorstehende Teil oder das hintere axiale Ende 10 an einem geeigneten Werkzeug befestigt werden kann, um die erforderliche Kraft ausüben zu können.

Nach einer anderen Ausführungsform der Erfindung sind die Vorrichtungen 8 nicht ausschliesslich dafür vorgesehen, dass darauf eine Rotationskraft, sondern zugleich eine Axialkraft ausgeübt werden kann und zwar sowohl eine Druck-, wie eine Zug-Kraft, damit, falls erforderlich, das Implantat 6 bei der Anbringung zwischen den Wirbelkörpern 1 und 2 eingedrückt und bei eventueller erneuter Entnahme, bei Verklemmung, eine Zugkraft darauf ausgeübt werden kann. Damit ist es jederzeit möglich, das Implantat 6 während des Eingriffs erneut zu entfernen.

Eine derartige Ausführungsform ist anhand der Fig. 9 dargestellt. Die Vorrichtungen 8 verbinden dabei ein erstes Ansatzelement 26, welches die Ausübung einer Rotationskraft zulässt, mit einem zweiten Ansatzelement 27, welches die Ausübung einer axialen Druck- und Zugkraft auf das Implantat 6 ermöglicht und dazu mit einer Axialsperre versehen ist.

Das erste Ansatzelement 26 besteht aus einer wie in der Ausführungsform nach Fig. 3 dargestellten Aussparung. Das zweite Ansatzelement 27 besteht aus einer zusätzlichen Aussparung, z.B. in Form eines Schlitzes in der Wand der obengenannten sechskantigen Öffnung, in den die Sperrelemente 28 des betreffenden Werkzeugs 9 greifen können. Wie in Fig. 9 dargestellt, können die Sperrelemente 28 aus Kugeln oder ähnlichem bestehen, die, nachdem das sechskantige Ende 11 des Werkzeugs 9 in die sechskantige Aussparung eingeführt wurde, radial nach aussen gedrückt werden und in den obengenannten Schlitz greifen.

Das Werkzeug 9 kann dabei verschiedene Formen aufweisen und auf unterschiedliche Art bedient werden. Gemäss Fig. 9 wird dies mit einem mit einem Keil 30 verbundenen Umschaltgriff 29 bewerkstelligt, der wiederum die Sperrelemente 28 auseinanderdrückt oder löst.

Bei einer anderen Variante ist das Schlüsselende gespalten, der Aussendurchmesser kann durch Andrücken oder Anschrauben eines inneren Stifts vergrössert werden, damit der Schlüssel in der Öffnung des Implantats 6, in das er eingeführt wird, eingeklemmt werden kann.

Nach einer anderen Variante können auch am vorderen axialen`'.Ende 12 mit der Abrundung 13 des Implantats 6 Ansatzmöglichkeiten für ein Werkzeug 9 vorgesehen werden. Diese Ansatzmöglichkeiten können von unterschiedlicher Art sein und sind vorzugsweise derart gestaltet, dass sie, gleich wie die Vorrichtungen 8, die Ausübung einer Rotationskraft, einer axialen Kraft und/oder einer seitlichen Kraft auf das Implantat 6 ermöglichen. Die Ansatzmöglichkeiten bestehen aus einer mehrkantigen, z.B. sechskantigen Öffnung, die die Anbringung eines Schlüssels mit entsprechendem Endstück ermöglicht, damit eine Torsionskraft auf das Implantat 6 ausgeübt werden kann, nachdem es nicht ausreichend fest angewachsen ist und auf abdominalem Weg entfernt werden muss. Diese Erfindung bezieht sich selbstverständlich auch auf Implantate 6, die an einem Ende mit Ansatzvorrichtung versehen sind, die eine Anbringung der Implantate auf abdominalem Weg ermöglichen.

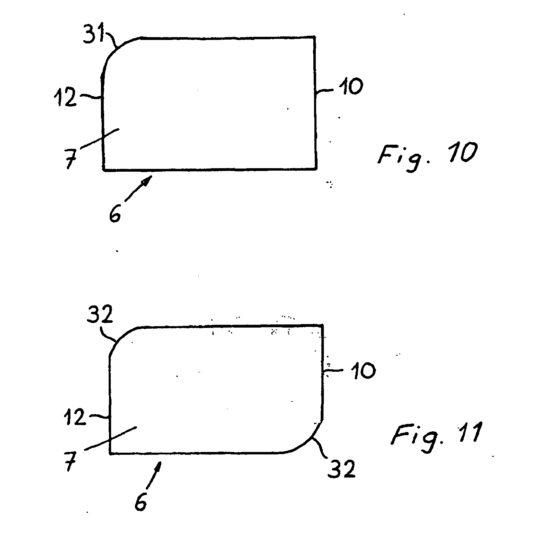

In den Fig. 10 und 11 sind weitere erfindungsgemässe Implantate dargestellt.
Fig. 10 zeigt den Körper 7 eines Implantates 6, welcher an der oberen Kante des vorderen axialen Endes 12 eine Abrundung 31 aufweist. Der Radius der einseitigen Abrundung 31 ist derart bemessen ist, dass a) die Differenz zwischen der grösseren Seite des rechteckigen Querschnittes und der Diagonale über die abgerundete Kante kleiner als 3 mm, vorzugsweise 1 - 2 mm beträgt; und b) die kleinere Fläche um weniger als die Hälfte, vorzugsweise um weniger als ein Drittel reduziert ist, d.h. die tragende Fläche sollte mindestens 2/3 der Gesamtbreite des Implantates entsprechen.

Fig. 11 zeigt den Körper 7 eines Implantates 6 im Querschnitt, wobei das Implantat im Querschnitt über die Diagonale je eine Abrundung 32 aufweist. Die Radien der gegenseitigen Abrundungen 32 sind derart bemessen, dass a) die Differenz zwischen der grösseren Seite des Querschnittes und der Diagonale über die abgerundeten Kanten kleiner als 3 mm, vorzugsweise 0,5 - 1,0 mm beträgt und b) die kleinere Fläche des Implantates um weniger als die Hälfte, vorzugsweise kleiner als ein Drittel reduziert ist.

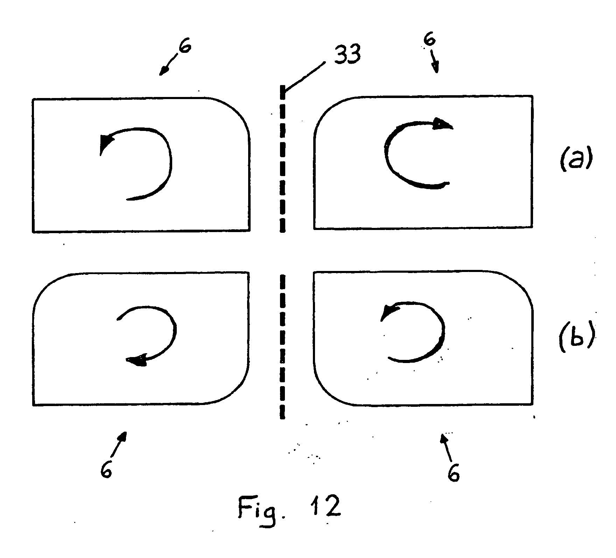

In Fig. 12 sind zwei bezüglich der Symmetrieachse 33 symmetrisch angeordnete Paare von Implantaten 6 dargestellt, wobei die beiden oberen Implantate 6 im Abschnitt (a) solche gemäss der Fig. 10 und die beiden unteren Implantate 6 im Abschnitt (b) solche gemäss der Fig. 11 darstellen.

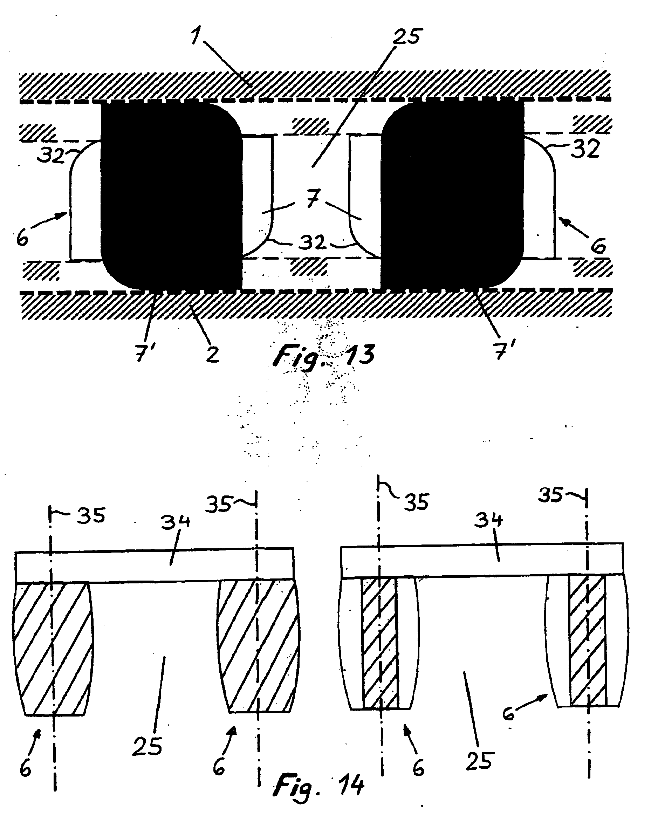

Beim Aufrichten (Rotieren) eines wie in Fig. 6 gezeigten Implantates 6 wird der Zwischenwirbelraum 25 um etwa 3-4 mm überdehnt, was zu einem Einbrechen der Deckplatten und permanenten Überdehnen des Bindegewebes führen kann. Wenn die Kanten nun eine Abrundung (31,32) aufweisen, wird die Überdehnung stark reduziert, damit ist aber auch die Stabilität der aufgerichteten Implantate reduziert oder die gepaarten Implantate sind spiegelsymmetrisch angeordnet um sich gegenseitig zu stabilisieren (siehe Fig. 13).

Die Aufrichtung mittels zweier Implantate 6, welche mit Abrundungen 31 oder 32 versehen sind führt bei geeignet gewählten Radien der Abrundungen 31 oder 32 zu einer Überdistrahierung des Zwischenwirbelraumes 25 von nur 1 mm; dafür sind aber die einzelnen aufgerichteten Implantate 6 nicht allzu stabil; sie können so leicht zurückkippen wie sie aufzurichten waren. In Fig. 13 schützen sich die beiden Implantate 6 durch die spiegelsymmetrische Geometrie gegenseitig vor dem Umkippen, da die Implantate 6 nur im Verbund und nicht einzeln umkippen können.

In Fig. 13 sind zwei spiegelsymmetrische angeordnete Implantate 6 gemäss Fig. 11 dargestellt. Die Abrundungen 32 der Körper 7 kommen dabei symmetrisch zueinander zu liegen. Die Körper 7 der Implantate 6 liegen nach deren Einführung horizontal zwischen den Wirbelkörper 1 und 2 und können dann mittels eines geeigneten Werkzeugs 9 um 90° in die schwarz gezeichnet Stellung 7' rotiert werden um den Zwischenwirbelraum 25 distrahieren zu können. Der rechteckige Querschnitt der Körper 7 ist derart beschaffen, dass nach der Rotation des Implantates 6 um 90° in die Konkavität der Deckplatten der angrenzenden Wirbelkörper 1,2 eine Distraktion des Zwischenwirbelraumes 25 zwischen 1 und 4 mm, vorzugsweise zwischen 2 - 3 mm, verbleibt.

In Fig. 14 sind zwei flach im Zwischenwirbelraum 25 (Zeichnungsebene) liegende Implantate 6 dargestellt, die über ein Konnektor 34 anterior miteinander verbunden sind. Im linken Teil der Fig. 14 ist die Stellung vor der Rotation der Implantate 6 dargestellt, im rechten Teil der Fig. 14 nach erfolgter Rotation um 90° in die Konkavität der Deckplatten der angrenzenden Wirbelkörper.

Das posteriore Ende der Implantate 6 bleibt frei und ist nur anterior durch den Konnektor 34 verbunden, so dass (a) der Abstand zwischen dem linken und rechten Implantat 6 und deren Ausrichtung zueinander aufrechterhalten wird, (b) die Implantate 6 um ihre Längsachse 35 drehbar sind, und (c) die beiden Implantate 6 vor deren Implantation und/oder in situ mit dem Konnektor 34 koppelbar sind.

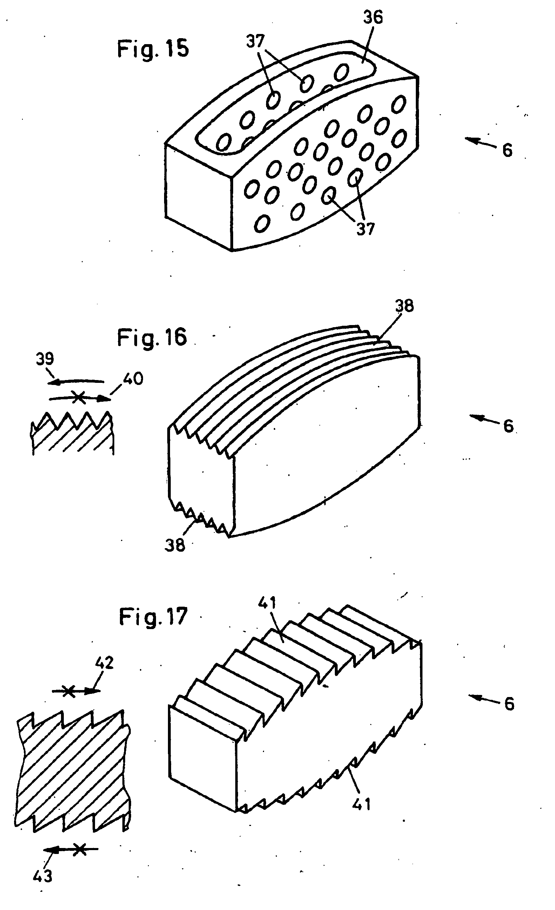

In Fig. 15 ist ein Implantat 6 mit einem Längsschnitt 35 zur Aufnahme von spongiösen Knochenmaterial oder osteokonduktives bzw. osteoinduktives Material und Querperforationen 36 der Wände für das Knochenwachstum dargestellt. Der Durchmesser der Perforationen 36 ist vorzugsweise derart konzipiert ist, dass (a) in die Längsöffnung 36 gepresste Spongiosa nicht seitlich austritt, und (b) die in der Spongiosa enthaltene Flüssigkeit beim Stopfen der Implantate 6 seitlich austreten und nach der Implantation wieder zurückdiffundieren kann, um ein postoperatives Schwellen der Spongiosa zu bewirken; und (c) Knochen durch die Perforationen 37 in das Implantat (6) hineinwachsen kann.

In Fig. 16 ist ein Implantat 6 dargestellt, dessen Kontaktflächen zwischen Implantat 6 und Knochen mit einer Längsstrukturierung 38 versehen ist. Die Längsstrukturierung 38 ist vorzugsweise derart gestaltet, dass das Rotieren des Implantates 6 in die Konkavität der Deckplatten nur in einer Richtung möglich ist, wie dies durch die Pfeile 39,40 angedeutet ist.

In Fig. 17 ist ein Implantat 6 dargestellt, dessen Kontaktflächen zwischen Implantat 6 und Knochen mit einer Querstrukturierung 41 versehen ist. Die Querstrukturierung 41 ist vorzugsweise derart gestaltet, dass die eine Kontaktfläche eine Translation in anteriorer und die andere eine Translation in posteriorer Richtung verhindert, wie dies durch die Pfeile 42,43 angedeutet ist. Die Verhinderung der Translation in anteriorer Richtung führt zu einer Entlastung des verbliebenen Annulus, der nach jüngster Forschung innerviert ist und damit auf anterioren Druck mit Schmerzsignalen reagieren könnte.

Die in den Figuren 15-17 schematisch dargestellten Implantate sind nicht erfindungsgemäss und dienen lediglich zur Illustrierung der möglichen Ausgestaltung der Kontaktflächen zum Knochen.

Diese Erfindung ist keinesfalls auf die als Beispiele gegebenen und in den Abbildungen dargestellten Modelle begrenzt - derartige Dilatatoren und das dazugehörende Werkzeug können in unterschiedlichen Formen und Grössen gefertigt werden, ohne dabei aus dem Rahmen der Definitionen, die in der Zusammenfassung im Anhang gegeben werden, zu fallen.

## Patentansprüche

1. Implantat (6) für den Zwischenwirbelraum (25) mit
A) einem im wesentlichen quaderförmigen Körper (7) mit verschiedenen Durchmessern (D1, D2) zwischen den Seitenflächen (14;15) sowie zwischen einer Oberfläche (16) und einer Unterfläche (17); wobei
B) die Seitenflächen (14;15), zwischen denen sich der kleinste Durchmesser (D1) erstreckt, grösstenteils parallel und flach sind; und mit
C) einer Vorrichtung (8) zur Ergreifung durch ein Werkzeug (9),
**dadurch gekennzeichnet, dass**
D) das Implantat (6) am Längsschnitt des grössten Durchmessers (D2) ein abgekantetes, linsenförmiges Profil aufweist;
E) die Oberfläche (16) und die Unterfläche (17) zwischen denen sich der grösste Durchmesser D2 erstreckt, zur Anlage an je einen der angrenzenden Wirbelkörper bestimmt sind;
F) das Implantat einen zur Längsachse (35) orthogonalen, teilweise abgerundeten Querschnitt aufweist; und
G) das Implantat (6) mit einer durchgehenden Öffnung (18) versehen ist, die die Form einer länglichen Rille mit parallelen Wänden (19, 20) aufweist. gemessene

2. Implantat (6) nach Anspruch 1, **dadurch gekennzeichnet, dass** mindestens eine seiner axialen Endflächen (10;12) abgerundet ist.

3. Implantat (6) nach einem der Ansprüche 1 bis 2, **dadurch gekennzeichnet, dass** es aus Titan oder einer Titanlegierung besteht.

4. Implantat (6) nach einem der Ansprüche 1 bis 3, **dadurch gekennzeichnet, dass** die Vorrichtung (8) derart beschaffen ist, dass sie die Ausübung einer Rotationskraft auf das Implantat (6) ermöglicht.

5. Implantat (6) nach einem der Ansprüche 1 bis 4, **dadurch gekennzeichnet, dass** die Vorrichtung (8) mindestens aus einer in der Innenseite des Körpers (7) vorgesehenen Aussparung besteht, in welche das vorzugsweise schlüsselähnliche Werkzeug (9) einführbar ist.

6. Implantat (6) nach einem der Ansprüche 1 bis 5, **dadurch gekennzeichnet, dass** die Vorrichtung (8) so gestaltet ist, dass mindestens die Ausübung einer Axial-, Druck- oder Zug-Kraft auf das Implantat (6) möglich ist.

7. Implantat (6) nach einem der Ansprüche 1 bis 6, **dadurch gekennzeichnet, dass** die Vorrichtung (8) so gestaltet ist, dass mindestens die Ausübung einer seitlichen Kraft auf das Implantat (6) möglich ist.

## Claims

1. Implant (6) for the inter-vertebral space (25) with
A) an essentially cuboid shaped body (7) with different diameters (D1, D2) between the lateral surfaces (14, 15) as well as between an upper surface (16) and a lower surface (17); whereby
B) the lateral surfaces (14, 15) with the smallest diameter (D1) extending between are mostly parallel and flat; and with
C) a device (8) for grasping by a tool (9),
**characterized in that**
D) the implant (6) presents a chamfered lenticular profile in the longitudinal section of its largest diameter (D2);
E) the upper surface (16) and the lower surface (17) with the largest diameter (D2) extending between are destined to contact an adjacent vertebral body each;
F) the implant presents a cross section orthogonal to the longitudinal axis which is partially rounded off; and
G) the implant (6) is provided with a continuous opening (18) which has the form of an elongated groove with parallel walls (19, 20).

2. Implant (6) according to claim 1, **characterized in that** at least one of its axial end surfaces (10, 12) is rounded off.

3. Implant (6) according to one of the claims 1 to 2, **characterized in that** it consists of titanium or a titanium alloy.

4. Implant (6) according to one of the claims 1 to 3, **characterized in that** the device (8) is configured so that it allows exerting a rotational force on the implant (6).

5. Implant (6) according to one of the claims 1 to 4, **characterized in that** the device (8) consists of at least one cavity provided inside the body (7), in which the preferably key-shaped tool (9) can be inserted.

6. Implant (6) according to one of the claims 1 to 5, **characterized in that** the device (8) is configured so that it allows exerting at least an axial-, pressing- or tensile force on the implant (6).

7. Implant (6) according to one of the claims 1 to 6, **characterized in that** the device (8) is configured so as to allow exerting at least a lateral force on the implant (6).

## Revendications

1. Implant (6) pour l'espace intervertébral (25), comprenant
A) un corps essentiellement parallélépipédique (7) avec différents diamètres (D1, D2) entre les faces latérales (14 ; 15) et entre une face supérieure (16) et une face inférieure (17) ; dans lequel
B) les faces latérales (14 ; 15), entre lesquelles s'étend le diamètre le plus petit (D1), sont essentiellement parallèles et planes ; et comprenant
C) un dispositif (8) pouvant être saisi par un outil (9),
**caractérisé en ce que**
D) l'implant (6) présente un profil lenticulaire chanfreiné relié au niveau de la coupe longitudinale du diamètre le plus grand (D2) ; dans lequel
E) la face supérieure (16) et la face inférieure (17) entre lesquelles s'étend le diamètre le plus grand (D2), sont destinées à être appuyées contre un des corps vertébraux adjacents ;
F) l'implant présente une coupe droite partiellement arrondie, orthogonale à l'axe longitudinal (35); et
G) l'implant (6) est pourvu d'une ouverture continue (18) qui a la forme d'une gorge longitudinale avec des parois parallèles (19, 20).

2. Implant (6) selon la revendication 1, **caractérisé en ce qu'**au moins une de ses faces d'extrémité axiales (10 ; 12) est arrondie.

3. Implant (6) selon l'une quelconque des revendications 1 ou 2, **caractérisé en ce qu'**il est fait de titane ou d'un alliage de titane.

4. Implant (6) selon l'une quelconque des revendications 1 à 3, **caractérisé en ce que** le dispositif (8) est conçu de telle manière qu'il permet l'application d'une force de rotation sur l'implant (6).

5. Implant (6) selon l'une quelconque des revendications 1 à 4, **caractérisé en ce que** le dispositif (8) est formé par au moins un évidement prévu dans la face interne du corps (7), dans lequel l'outil (9), de préférence analogue à une clé, peut être inséré.

6. Implant (6) selon l'une quelconque des revendications 1 à 5, **caractérisé en ce que** le dispositif (8) est conçu de telle manière qu'au moins l'application d'une force axiale, d'une force de compression ou d'une force de traction sur l'implant (6) est possible.

7. Implant (6) selon l'une quelconque des revendications 1 à 6, **caractérisé en ce que** le dispositif (8) est conçu de telle manière qu'au moins l'application d'une force latérale sur l'implant (6) est possible.
